(19) Europäisches Patentamt
European Patent Office
Office européen des brevets

(11) EP 0 571 520 B1

(12) **FASCICULE DE BREVET EUROPEEN**

(45) Date de publication et mention de la délivrance du brevet: **10.09.1997 Bulletin 1997/37**

(21) Numéro de dépôt: **92906691.8**

(22) Date de dépôt: **14.02.1992**

(51) Int. Cl.[6]: **A61K 31/35**, A61K 31/765, A61K 35/78

(86) Numéro de dépôt international: **PCT/FR92/00147**

(87) Numéro de publication internationale: **WO 92/14457 (03.09.1992 Gazette 1992/23)**

(54) **UTILISATION EN THERAPEUTIQUE DE PYCNOGENOLS POUR LA PREPARATION DE MEDICAMENTS A ACTIVITE ANTI-INFLAMMATOIRE**

THERAPEUTISCHE VERWENDUNG VON PYCNOGENOLEN ZUR HERSTELLUNG EINES MEDIKAMENTES MIT ANTIINFLAMMATORISCHER WIRKUNG

NOVEL THERAPEUTIC USE OF PYCNOGENOLS FOR THE PREPARATION OF ANTI-INFLAMMATORY MEDICAMENTS

(84) Etats contractants désignés: **AT BE CH DE DK ES FR GB GR IT LI LU MC NL SE**

(30) Priorité: **15.02.1991 FR 9101842**

(43) Date de publication de la demande: **01.12.1993 Bulletin 1993/48**

(73) Titulaire: **LABORATOIRES DOLISOS**
**75003 Paris (FR)**

(72) Inventeurs:
- **TITS, Monique**
  **B-4041 Votten (BE)**
- **ANGENOT, Luc**
  **B-4400 Flemalle (BE)**

(74) Mandataire: **Gillard, Marie-Louise**
**Cabinet Beau de Loménie**
**158, rue de l'Université**
**75340 Paris Cédex 07 (FR)**

(56) Documents cités:
**DE-A- 2 740 346** **FR-A- 2 092 743**
**FR-A- 2 624 737**

- **Planta Medica, vol. 57, suppl. 2, décembre 1991; M. TITS et al.: "Anti-inflammatory prodelphinidins from black currant (Ribes nigrum) leaves", p. A134**
- **Journal of Ethnopharmacology, vol. 27, no. 1, novembre 1989, (IE); C. DECLUME: "Anti-inflammatory evaluation of a hydroalcoholic extract of black currant leaves (Ribes nigrum)", pages 91-98**
- **Acta Physiologica Acad. Sci. Hung., vol. 56, no. 2, 1980; G. BLAZSO et al.: "Oedema-inhibiting effect of procyanidin", pages 235-240**
- **"Dictionnaire Vidal", 63 édition, 1987, page 1690, Paris, FR, page 1690**
- **Phytochemistry, vol. 14, 1975, (GB); E.C. BATE-SMITH: "Phytochemistry of proanthocyanidins", pages 1107-1113**
- **Bulletin Officiel n 90/22bis du ministères des affaires sociales et de la solidarité (1990), Médicaments à base de plantes, p.24-31**
- **Ethnopharmacologie (1990), Actes du 1er colloque européen d'éthnopharmacologie, Metz 22-25/3/90, p.406-8**
- **Ressources Médicinales de la Flore française (1961), T.II, Vigot Frères éd., Garnier et al, p.707-8**
- **Acta Physiol. Acad. Sci. Hung. (1980), vol.56, p.235-40**

Remarques:
Le dossier contient des informations techniques présentées postérieurement au dépôt de la demande et ne figurant pas dans le présent fascicule.

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

Printed by Rank Xerox (UK) Business Services
2.14.14/3.4

EP 0 571 520 B1

## Description

L'invention concerne une nouvelle utilisation en thérapeutique de pycnogénols pour la préparation de médicaments à activité anti-inflammatoire.

L'invention concerne plus particulièrement une nouvelle utilisation en thérapeutique de pycnogénols contenus dans des extraits de Ribes nigrum ou isolés et purifiés à partir de tels extraits.

De nombreux végétaux sont connus pour leur activité anti-inflammatoire ou des activités apparentées, telle que l'activité anti-rhumatismale. C'est le cas en particulier de la variété Ribes nigrum, de la famille des Saxifragaceae. L'activité anti-inflammatoire d'extraits hydroalcooliques totaux de feuilles de Ribes nigrum ou de leurs lyophilisats administrés par voie orale a été récemment rapportée par C. Declume dans J. Ethnopharmacol., 1989, 27, 91-98

La publication C. Declume, Ethnopharmacologie, Premier colloque européen d'éthnopharmacologie, Metz 23-25/03/90, Société française d'éthnopharmacologie, ORSTOM Editions, p. 406-408, concerne l'étude comparative de la réponse anti-inflammatoire obtenue avec différentes formes médicinales à base d'extraits de Ribes nigrum.

Des compositions pharmaceutiques à base d'anthocyanidines, pour lesquelles une activité anti-inflammatoire est mentionnée, ainsi que leur procédé d'obtention sont décrits dans la demande FR 2 364 030.

L'activité des anthocyanidines extraites de dits vis-à-vis de la perméabilité capillaire est mentionnée dans le Bulletin Officiel n° 90/22bis du Ministère des Affaires Sociales et de la Solidarité, 1990, Médicaments à base de plantes, p. 24-31.

L'article G. Blazso et al, Acta Physiologica. Acad. Sci-Hung, 1980, 56, 235-240, concerne l'étude de l'effet inhibiteur d'oedème de la procyanidine extraite de raisins.

Bien que l'activité thérapeutique de tels extraits soit connue, le ou les principe(s) actif(s) responsable(s) de l'activité anti-inflammatoire ou d'activités apparentées n'ont pas encore été identifiés.

On a maintenant trouvé, de manière surprenante, que l'activité anti-inflammatoire d'extraits de Ribes nigrum était essentiellement due à la présence de pycnogénols dans ces extraits. Les pycnogénols sont des composés constitués d'unités flavane-3-ol qui sont présents dans divers végétaux. L'unité de base de ces polymères est le plus souvent la catéchine : on appelle alors ces polymères des procyanidines.

Des procédés permettant la préparation d'extraits contenant des pycnogénols ont été décrits dans les brevets GB 1 541 469 et FR 2 092 743.

Par ailleurs, la demande EP 275 224 décrit la préparation de complexes phospholipidiques d'extraits de Vitis vinifera, ces derniers étant constitués d'oligoméres procyanidoliques, ainsi que des compositions pharmaceutiques à activité protectrice vasculaire et des compositions cosmétiques contenant lesdits complexes.

L'invention concerne donc l'utilisation de pycnogénols de formule (I) :

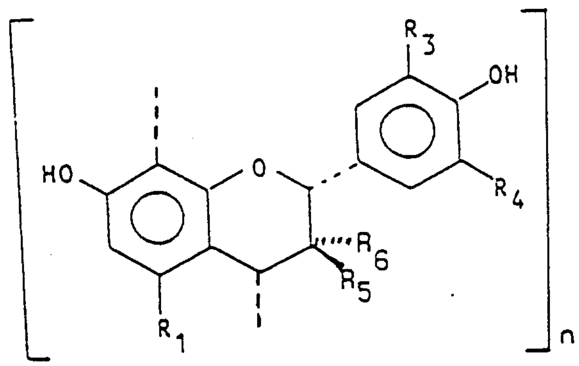

(I)

dans laquelle

- $R_1$, $R_3$ et $R_4$ représentent OH,
- $R_5$ et $R_6$ sont différents et représentent H ou OH, et
- n est un nombre entier de 2 à 40,

lesdits pycnogénols étant soit contenus dans des extraits de Ribes nigrum à raison d'au moins 2 % en poids, soit sous forme isolée et purifiée à partir d'extraits de Ribes nigrum, pour la préparation de médicaments à activité anti-inflammatoire. Notamment, on extrait les pycnogénols de formule (I) à partir des feuilles ou des bourgeons de Ribes nigrum.

Avantageusement, on a pu montrer par l'analyse chimique des extraits de Ribes nigrum les plus actifs sur le plan

pharmacologique que l'unité de base des pycnogénols présents dans ces extraits était le plus souvent la gallocatéchine, et non la catéchine. De tels polymères sont appelés prodelphinidines.

L'invention concerne donc l'utilisation de prodelphinidines, c'est-à-dire de pycnogénols de formule (I) telle que définie ci-dessus, lesdits pycnogénols étant soit contenus dans des extraits de Ribes nigrum à raison d'au moins 2 % en poids, soit sous forme isolée et purifiée à partir d'extraits de Ribes nigrum, pour la préparation de médicaments à activité anti-inflammatoire. Les prodelphinidines se différencient des oligomères procyanidoliques par le fait que le substituant $R_4$ représente OH dans le cas des prodelphinidines, alors qu'il représente H dans le cas des procyanidines.

La préparation des pycnogénols utilisables selon l'invention peut êre résumée en deux étapes principales :

1) Préparation d'un extrait total selon les techniques usuelles d'extraction végétale, telles que, par exemple, les extractions aqueuse, hydroalcoolique ou hydroacétonique ;
2) Isolement et purification des pycnogènols.

Dans un procédé avantageux pour préparer un extrait de Ribes nigrum (1ère étape), on pulvérise un lot de feuilles que l'on épuise par percolation lente au moyen d'éthanol à 70°. L'éthanol est éliminé par distillation à l'évaporateur rotatif, sous pression réduite, à une température inférieure à 50°C. La solution aqueuse restante est filtrée afin d'éliminer une partie importante de chlorophylle. On pratique ensuite un fractionnement en utilisant successivement des solvants de polarité croissante, tels que :

a) l'éther diéthylique afin d'éliminer la chlorophylle restante et diverses substances liposolubles ;
b) l'acétate d'éthyle ;
c) le n-butanol.

Dans un autre procédé avantageux d'extraction, on épuise un lot de poudre de feuilles sèches de Ribes nigrum par percolation lente, au moyen d'un mélange acétone-eau. Après obtention du dernier percolat, on exprime la poudre à la presse et on rassemble tous les liquides. L'extrait total est agité vigoureusement avec du NaCl, ajouté jusqu'à l'obtention de deux phases : une phase supérieure acétonique et une phase inférieure aqueuse. La phase acétonique est évaporée sous pression réduite à 30°C et le résidu aqueux est additionné d'un égal volume d'eau, filtré puis ré-extrait : soit par de l'acétate d'éthyle, soit par du butanol. Les extraits sont ensuite évaporés à sec.

On obtient ainsi des extraits contenant au moins 2% en poids de pycnogénols de formule (I).

L'isolement et la purification des pycnogénols utilisables selon l'invention (2e étape) peuvent être réalisés par différentes techniques de chromatographie sur gel, ou encore des techniques d'ultra-filtration connues de l'homme du métier.

Parmi les techniques chromatographiques, on citera notamment l'utilisation de la colonne Fractogel TSK (MERCK, USA) en éluant à l'aide d'eau, puis d'un mélange eau/méthanol à pourcentage croissant en méthanol ; la colonne Sephadex® LH 20 (PHARMACIA, SUEDE) en éluant à l'aide d'alcool puis d'alcool additionné d'eau ; ou encore sur une colonne Lobar Lichroprep RP8 (MERCK, USA), l'élution étant réalisée à l'aide d'un mélange eau/acétone. Ces différentes techniques peuvent être combinées entre elles pour obtenir un plus haut degré de purification.

Ceci permet d'obtenir les pycnogénols de formule (I) sous forme isolée et purifiée.

Les pycnogénols isolés et purifiés ont été soumis à différentes techniques spectrales en vue de déterminer leur structure.

On a ainsi pu mettre en évidence des pycnogénols particuliers sous forme de dimères de gallocatéchine. Ces molécules répondent à la formule (I) :

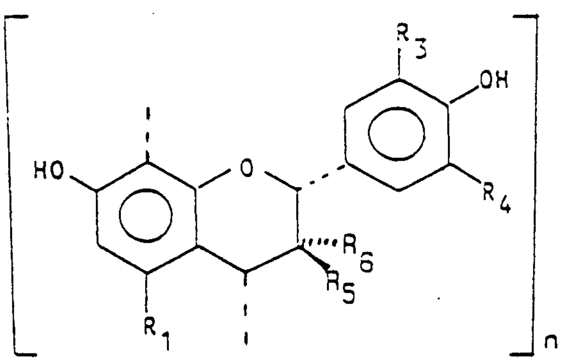

(I)

dans laquelle

- $R_1$, $R_3$ et $R_4$ représentent OH,
- $R_5$ et $R_6$ sont différents et représentent H ou OH, et
- n = 2.

D'autres pycnogénols particuliers se présentent sous forme de trimères de gallocatéchine, c'est-à-dire de composés de formule (I) :

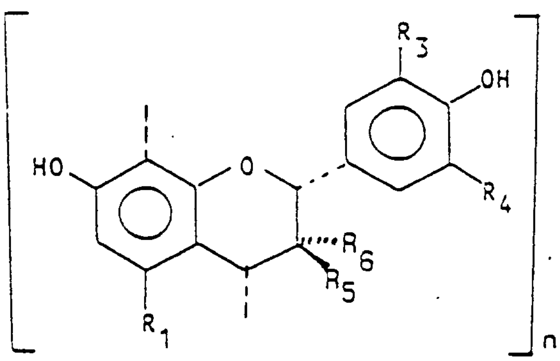

(I)

dans laquelle

- $R_1$, $R_3$ et $R_4$ représentent OH,
- $R_5$ et $R_6$ sont différents et représentent H ou OH, et
- n = 3.

L'utilisation de ces pycnogénols particuliers sous forme de dimères ou de trimères de gallocatéchine ainsi que leurs isomères pour la préparation de médicaments anti-inflammatoires représente un aspect préféré de la présente invention.

Dans un aspect avantageux, on utilisera des dimères ou des trimères de gallocathéchine dans lesquels $R_5$ représente OH et $R_6$ représente H.

Dans un autre aspect préféré, on utilisera des dimères ou des trimères de gallocathéchine dans lesquels $R_5$ représente H et $R_6$ représente OH.

L'activité anti-inflammatoire des pycnogénols extraits de Ribes nigrum tels que décrits plus haut a été testée notamment à l'aide du test de l'oedème à la carraghénine tel que décrit dans WINTER et al., Exp. Biol. Med., 1962, 111, 544-547. Le principe de ce test consiste à provoquer un gonflement aigü de la patte d'un rat par une injection de carraghénine.

L'évaluation de l'activité anti-inflammatoire des composés étudiés est basée sur leur pouvoir de réduire cet oedème par comparaison à des animaux témoins traités par le seul solvant. La mesure est effectuée en comparant, à différents temps, le volume de la patte ayant reçu la carraghénine à celui de la patte controlatérale ayant reçu le même volume de sérum physiologique.

Les pycnogénols de formule (I) contenus dans des extraits de Ribes nigrum à raison d'au moins 2% en poids ou sous forme isolée et purifiée ont montré une activité significative dans ce test.

Les composés de formule (I) peuvent être utilisés pour la préparation de médicaments à activité anti-inflammatoire, caractérisés en ce qu'ils contiennent un ou plusieurs composés de formule (I) contenus dans des extraits de Ribes nigrum à raison d'au moins 2% en poids ou sous forme isolée et purifiée en tant que principe actif en association avec un véhicule pharmaceutique.

Avantageusement, lesdits médicaments à activité anti-inflammatoire contiendront une quantité standardisée de pycnogénols de formule (I).

L'invention concerne notamment l'utilisation des composés de formule (I) pour la préparation de médicaments à activité anti-inflammatoire sous une forme convenant pour l'administration par voie orale, telle que comprimé, gélule ou pilule.

Les pycnogénols de formule (I) peuvent également être utilisés pour la préparation de médicaments à activité anti-inflammatoire sous une forme convenant pour l'administration parentérale, notamment par voie intraveineuse, sous-cutanée ou intra-musculaire ou encore sous une forme convenant pour l'administration locale ou rectale.

Notamment, les pycnogénols de formule (I) seront utilisés dans des compositions pour administration par voie orale sous forme de complexes liposolubles.

Pour l'administration par voie digestive (orale ou rectale) on peut avantageusement associer les pycnogénols de formule (I) à un dérivé d'anthraquinone améliorant le passage à travers la muqueuse digestive, en particulier l'istizine (1,8-dihydroxyanthraquinone).

La quantité de principe actif à administrer dépendra de la nature et de la sévérité de l'inflammation, ainsi que du poids du patient et de la voie d'administration.

A titre indicatif, les doses seront de l'ordre de 500 mg/j pour l'administration par voie parentérale, et de l'ordre de 1,5 g/j pour l'administration orale.

Les pycnogènols de formule (I) peuvent également constituer des composés marqueurs de l'activité anti-inflammatoire dans des extraits végétaux. Par "composé marqueur" on entend un composé dont la présence est indicatrice de l'activité recherchée. Dans un aspect avantageux, l'invention concerne donc également un procédé de détermination de l'activité anti-inflammatoire dans un extrait végétal, caractérisé en ce qu'on effectue la détection et le dosage des pycnogénols de formule (I) dans cet extrait.

Pour détecter et doser les pycnogénols de formule (I) totaux dans un extrait végétal, on utilisera avantageusement un dosage spectrophotométrique en lumiere visible, par mesure de l'absorbance de la delphinidine formée lors du chauffage en milieu butanol/HCl.

A titre d'exemple, on prépare un extrait de 50 mg en utilisant les techniques décrites plus haut, que l'on solubilise dans 10 ml de méthanol. On pipette 5 ml de cette solution à laquelle on ajoute dans un ballon de 50 ml, une solution de butanol contenant 5 ml de HCl. On chauffe à reflux à 100|C pendant 2 heures puis on transfère le contenu du ballon dans un ballon jaugé à 50 ml et on amène au trait à l'aide de butanol, ayant servi au préalable à rincer le ballon. On mesure l'absorbance de cette solution à 558 nm sous une épaisseur d'un cm en prenant l'eau comme liquide de compensation.

On peut également effectuer un dosage gravimétrique en calculant la quantité de pycnogénols retenus sur de la poudre de peau.

A titre d'exemple, l'extraction de ces substances peut se faire suivant le mode opératoire décrit plus haut pour la chromatographie en utilisant 2 g de feuilles, l'extrait étant amené en fin d'opération à 100 ml avec de l'eau.

- 25,0 ml du filtrat sont évaporés à sec à l'évaporateur rotatif et pesés suivant des conditions opératoires bien précises. Ce résidu correspond au résidu total R1.
- A 50,0 ml de filtrat restant, on ajoute 1 g de poudre de peau faiblement chromée (ref. 4352, MERCK, USA) et on agite énergiquement pendant 1 heure. On filtre sur filtre serré et on évapore à sec 25,0 ml de ce filtrat dans les mêmes conditions que ci-dessus. Ce résidu correspond aux substances non retenues sur la poudre de peau = R2.

Le pourcentage de pycnogénols adsorbés sur la poudre de peau se calcule en appliquant la formule :

$$\frac{(R1 - R2) \times 400}{pg}$$

dans laquelle pg représente la quantité de feuilles mise en oeuvre.

La détection et le dosage de pycnogénols de formule (I) déterminés, tels que notamment le dimère et le trimère de gallocatéchine décrits plus haut, sera avantageusement effectuée par chromatographie sur couche mince ou par HPLC (chromatographie liquide haute performance).

A titre d'exemple de protocole utilisable en chromatographie sur couche mince, on peut effectuer une extraction sur 2 g de feuilles pulvérisées laissées en contact pendant 2 heures avec quelques ml de mélange acétone - eau (7 : 3). Puis on percole avec ce même mélange jusqu'à obtenir 25 ml de solution. Cette solution est évaporée sous pression réduite à une température inférieure à 45°C. Le résidu est repris par 10 ml de mélange méthanol - eau (1 : 1). On dépose 10 µl de cette solution sur une plaque de silicagel en utilisant la phase supérieure d'un mélange acétate d'éthyle - eau - ac.formique - ac.acétique (70:20:3:2) pour le développement. La révélation est effectuée par une solution à 1 % de vanilline dans HCl/méthanol (1:1). Les pycnogénols se présentent à la lumière du jour sous forme de plusieurs bandes rouges. La zone des Rf les plus élevés correspond aux monomères ; une bande importance se situant vers le milieu du chromatogramme correspond aux dimères de la gallocatéchine. Il apparaît d'autres bandes rouges à des Rf inférieurs correspondant aux molécules plus polymérisées.

Pour étudier des extraits par la méthode HPLC, on peut par exemple utiliser un appareil Diode-Array (PHARMACIA, SUEDE ) couplé à un microordinateur PC MEMOREX, en utilisant une colonne C18 de 25 cm (PHARMACIA, SUEDE).

L'invention sera mieux comprise à l'aide des exemples illustratifs ci-après, qui ne présentent aucun caractère limitatif.

Exemple 1 : Mise en évidence de l'activité anti-inflammatoire des pycnogénols par voie intrapéritonéale.

1 kg de feuilles de Ribes nigrum est pulvérisé et épuisé par percolation lente au moyen de 10 l d'éthanol à 70°. L'éthanol est éliminé par distillation à l'évaporateur rotatif, sous pression réduite, à une température inférieure à 50°C. La solution aqueuse restante est filtrée afin d'éliminer une partie importante de chlorophylle. Le tiers de la solution aqueuse est lyophilisé tel quel et conservé comme référence = extrait brut (70 g).

Sur les deux-tiers restants, on pratique un fractionnement en utilisant successivement des solvants de polarité croissante et en soumettant à chaque étape la solution résiduelle à un nouveau fractionnement :

à 10 ml de solution aqueuse, on ajoute 30 ml d'éther diéthylique afin d'éliminer par décantation la chlorophylle restante et diverses substances liposolubles. On ajoute ensuite à la solution 30 ml d'acétate d'éthyle. Après décantation et évaporation, le résidu est récupéré et séché. 30 ml de n-butanol sont ensuite ajoutés à la solution aqueuse restante. Le résidu est récupéré et séché. La solution aqueuse résiduelle donne, après lyophilisation, un résidu correspondant à environ 11% du produit de départ.

L'activité anti-inflammatoire de ces différents extraits a été testée dans le test de l'oedème à la carraghénine tel que décrit par DAMAS (Thèse d'Agrégation, Université de Liège, 1981).

Les extraits recueillis aux différentes étapes de l'extraction sont diluées à l'eau au 1/10 puis lyophilisés.

On injecte par voie intrapéritonéale à 6 rats Winstar (pesant environ 240 g) 0,5 ml d'extrait lyophilisé dissous dans du sérum physiologique aux doses de 50 et 250 mg/kg. Une demi-heure après, on injecte dans le 2ème expace intermétatarsien d'une patte postérieure 0,1 ml d'une solution à 1 % de carraghénine Lambda pure (SIGMA, USA), la patte controlatérale recevant le même volume de sérum physiologique.

Le volume de chaque patte est mesuré à différents temps (2, 3 et 4 h) à l'aide d'un pléthysmomètre (UGOBASILE, ITALIE) et les résultats sont exprimés en pourcentage d'inhibition de l'oedème par rapport aux animaux témoins ayant reçu 0,5 ml de sérum physiologique.

Les résultats sont rapportés dans le tableau 1 ci-après :

Tableau 1

| | % d'inhibition à 50 mg/kg | % d'inhibition à 250 mg/kg |
|---|---|---|
| Extrait total | 33% (a) | 68% (a) |
| Extrait acétate d'éthyle | 16% (b) | 65% (a) |
| Extrait butanolique | 35% (c) | 92% (a) |
| Solution aqueuse restante | 46% (c) | 94% (a) |

(a) $p > 0,001$
(b) $p > 0,05$
(c) $p > 0,005$

Ces résultats montrent que l'extrait butanolique et la solution aqueuse restante présentent les activités les plus intéressantes.

Afin de déterminer la nature des molécules responsables de l'activité anti-inflammatoire, on fait réagir une solution aqueuse contenant 3 g d'extrait aqueux avec 5 g de poudre de peau standardisée faiblement chromée (référence 4332, MERCK, USA) sous agitation constante, pendant 1 h. En effet, les pycnogénols ont une très grande affinité pour les protéines et forment avec ces dernières différents types de complexes.

On réalise alors un essai comparatif entre la solution aqueuse précédemment testée et un échantillon de solution aqueuse passée sur poudre de peau, à la dose de 200 mg/kg, dans le modèle expérimental décrit plus haut. Les résultats sont rapportés dans le tableau 2 ci-après.

Tableau 2

| | % d'inhibition à 200 mg/kg |
|---|---|
| Solution aqueuse | 62% $p > 0,001$ |
| Solution aqueuse passée sur poudre de peau | 29% $p > 0,005$ |

Ces résultats montrent que l'activité de l'extrait aqueux est considérablement réduite lorsque la majorité des pycnogènols présents en a été éliminée.

On peut donc relier l'activité anti-inflammatoire en grande partie à la présence de pycnogénols dans les extraits de Ribes nigrum.

Exemple 2 : Mise en évidence de l'activité anti-inflammatoire de pycnogénols purifiés par voie intrapéritonéale.

On prépare un extrait total de Ribes nigrum en utilisant la méthode suivante :

500 g de feuilles broyées sont mis à macérer dans un mélange eau/acétone (rapport 7/3) pendant 12 h, puis la préparation subit une percolation lente permettant d'obtenir 5 kg de solution. Après pressage des feuilles, une nouvelle percolation est effectuée avec le mélange eau/acétone, suivie d'une distillation sous vide. Après évaporation de l'acétone, la solution est filtrée pour éliminer la chlorophylle précipitée. Le résidu aqueux est réextrait 5 fois consécutivement par 300 ml de n-butanol. Les extraits obtenus sont ensuite évaporés à sec et réunis.

On effectue une chromatographie préparative basse pression de cet extrait, sur colonne Lobar Lichroprep RP8 (MERCK, USA) en éluant avec un mélange eau + 10% d'acétone, en augmentant progressivement le pourcentage d'acétone. On obtient ainsi :

1) une fraction très polaire ne contenant pas de pycnogénols et précipitant en présence de méthanol ;
2) une fraction contenant différents pycnogénols et des acides phénols ;
3) une fraction renfermant surtout des flavonoïdes et les tanins catéchiques les plus polymérisés.

En faisant repasser la fraction 2 sur colonne Sephadex $^{R}$ LH20 (PHARMACIA, SUEDE) en éluant au moyen d'alcool, puis d'un mélange hydroalcoolique contenant 10, 20 puis 50% d'eau, on obtient des molécules purifiées qui ont été caractérisées comme des pycnogénols constitués respectivement d'un dimère et d'un trimère de gallocatéchine.

Différentes fractions ainsi que des molécules purifiées ont été soumises au test de l'oedème à la carraghénine décrit dans l'exemple 1 :

- une fraction provenant de la chromatographie sur Lobar RP8 et ne renfermant ni pycnogénols, ni flavonoïdes mais des substances solubles dans l'eau et précipitant par le méthanol (fraction 1) ;
- une fraction contenant presque exclusivement des acides phénols (fraction 2) ;
- l'isoquercitrin, flavonoïde majoritaire des feuilles ;
- le dimère et le trimère de la gallocatéchine.

Les résultats sont rapportés dans le tableau 3 ci-après :

Tableau 3

| Fractions ou molécules testées | Concentration mg/kg | Pourcentage d'inhibition | |
|---|---|---|---|
| | | après 2 h | après 4 h |
| Fraction 1 | 100 | n.s. | n.s. |
| Fraction 2 | 100 | n.s. | n.s. |
| Isoquercitrin | 100 | n.s. | n.s. |
| Dimère | 5 | 18 | 19 |
| | 10 | 37 | 7 |
| | 40 | 45 | 29 |
| Trimère | 5 | 18 | 15 |
| | 10 | 35 | 30 |
| | 40 | 58 | 50 |
| n.s. : non significatif | | | |

Ces résultats montrent que les molécules isolées présentent une inhibition de l'oedème à la carraghénine de l'ordre de 45 à 60% à la dose de 40 mg/kg.

Exemple 3 : Mise en évidence de l'activité anti-inflammatoire des pycnogénols par voie rectale.

On utilise un extrait brut acétonique préparé selon la méthode décrite dans l'exemple 2 en s'arrêtant avant l'étape de chromatographie préparative.

Pour améliorer le passage des pycnogénols à travers la muqueuse digestive, l'extrait de Ribes nigrum est associé à un dérivé des anthraquinones, l'istizine (1,8-dihydroxyanthraquinone).

1 mg d'istizine est dissous dans de l'alcool puis ajouté à raison de 0,01% à l'extrait acétonique de Ribes nigrum. Le mélange est assuré par sonication pendant 4 mn. Les rats sont anesthésiés à l'éther, un cathéter est ensuite introduit jusqu'à 6-7 cm du sphincter anal et la préparation administrée à raison de 0,5ml par rat (concentration de la solution : 50 mg/ml). L'injection de la carraghénine est pratiquée une heure après l'administration rectale de l'extrait. La mesure de l'oedème est effectuée 3 heures après l'injection. Les résultats, exprimés en pourcentage d'augmentation du volume de la patte par rapport au volume initial, sont rapportés dans le tableau 4 ci-après :

Tableau 4

| | Pourcentage d'augmentation du volume de la patte |
|---|---|
| Animaux traités ( n = 6) | 30,1 + 5,3 |
| Animaux témoins ( n = 6) | 77,8 + 8,3 |

Ces résultats montrent que l'association de pycnogénols et d'un dérivé des anthraquinones, l'istizine, induit une inhibition significative de l'oedème à la carraghénine de 61% ($p < 0,01$) pour une dose d'environ 100 mg/kg d'extrait.

## Revendications

**1.** Utilisation de pycnogénols de formule (I)

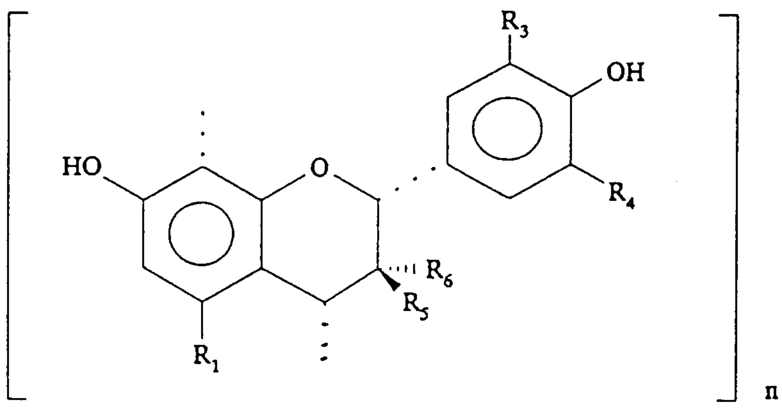

dans laquelle

- $R_1$, $R_3$ et $R_4$ représentent OH,
- $R_5$ et $R_6$ sont différents et représentent H ou OH, et
- n est un nombre entier de 2 à 40, lesdits pycnogénols étant soit contenus dans des extraits de Ribes nigrum à raison d'au moins 2% en poids, soit sous forme isolée et purifiée à partir d'extraits de Ribes nigrum, pour la préparation de médicaments à activité anti-inflammatoire.

2. Utilisation selon la revendication 1, caractérisée en ce que les pycnogénols répondent à la formule (I) dans laquelle $R_1$, $R_3$, $R_4$, $R_5$ et $R_6$ sont tels que définis dans la revendication 1 et n = 2.

3. Utilisation selon la revendication 1, caractérisée en ce que les pycnogénols répondent à la formule (I) dans laquelle $R_1$, $R_3$, $R_4$, $R_5$ et $R_6$ sont tels que définis dans la revendication 1 et n = 3.

4. Utilisation selon l'une des revendications 2 ou 3, caractérisée en ce que $R_5$ représente OH et $R_6$ représente H.

5. Utilisation selon l'une des revendications 2 ou 3, caractérisée en ce que $R_5$ représente H et $R_6$ représente OH.

6. Utilisation selon l'une quelconque des revendications 1 à 5, caractérisée en ce que pour l'administration par voie digestive, on associe les pycnogénols de formule (I) à un dérivé d'anthraquinone, notamment la 1,8-dihydroxyanthraquinone.

7. Procédé de détermination de l'activité anti-inflammatoire dans un extrait végétal, caractérisé en ce qu'il consiste à doser les pycnogénols de formule (I) dans cet extrait.

## Claims

1. Use of pycnogenols of formula (I)

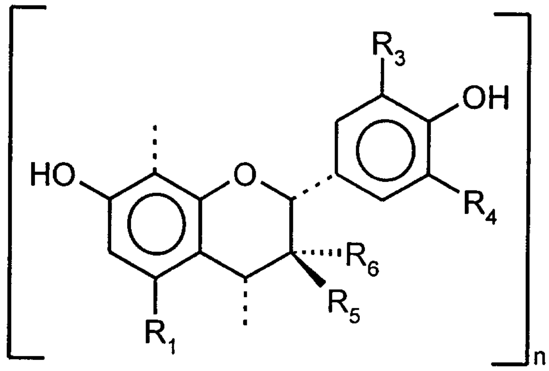

in which

- $R_1$, $R_3$ and $R_4$ represent OH,
- $R_5$ and $R_6$ are different and represent H or OH, and
- n is an integer from 2 to 40,

said pycnogenols being either contained in Ribes nigrum extracts at the rate of at least 2% by weight, or in the isolated and purified form from Ribes nigrum extracts, for the preparation of anti-inflammatory medicaments.

2. Use according to claim 1, characterised in that the pycnogenols are of formula (I) in which $R_1$, $R_3$, $R_4$, $R_5$ and $R_6$ are such as defined in claim 1 and n = 2.

3. Use according to claim 1, characterised in that the pycnogenols are of formula (I) in which $R_1$, $R_3$, $R_4$, $R_5$ and $R_6$ are such as defined in claim 1 and n = 3.

4. Use according to one of claims 2 or 3, characterised in that $R_5$ represents OH and $R_6$ represents H.

5. Use according to one of claims 2 or 3, characterised in that $R_5$ represents H and $R_6$ represents OH.

6. Use according to any one of claims 1 to 5, characterised in that for the administration via the digestive route, the pycnogenols of formula (I) are associated with an anthraquinone derivative, notably 1,8-dihydroxyanthraquinone.

7. Method of determination of the anti-inflammatory activity in a vegetable extract, characterised in that it consists in determining the quantity of pycnogenols of formula (I) in this extract.

**Patentansprüche**

1. Verwendung von Pygnogenolen der Formel (I)

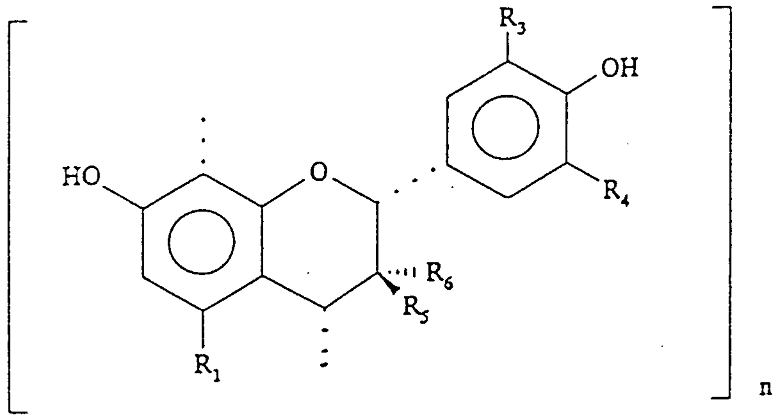

worin bedeuten

- $R_1$, $R_3$ und $R_4$ OH,
- $R_5$ und $R_6$, die verschieden sind, H oder OH, und
- n eine ganze Zahl im Bereich von 2 bis 40,

wobei die Pygnogenole entweder in einem Mengenanteil von mindestens 2 Gew.-% in Extrakten von Ribes nigrum enthalten sind oder in aus Extrakten von Ribes nigrum isolierter und gereinigter Form vorliegen, zur Herstellung von Arzneimitteln mit entzündungshemmender Wirksamkeit.

2. Verwendung nach Anspruch 1, dadurch gekennzeichnet, daß die Pygnogenole der Formel (I) entsprechen, worin $R_1$, $R_3$, $R_4$, $R_5$ und $R_6$ die in Anspruch 1 angegebenen Bedeutungen aufweisen und n 2 ist.

3. Verwendung nach Anspruch 1, dadurch gekennzeichnet, daß die Pygnogenole der Formel (I) entsprechen, worin $R_1$, $R_3$, $R_4$, $R_5$ und $R_6$ die in Anspruch 1 angegebenen Bedeutungen aufweisen und n 3 ist.

4. Verwendung nach einem der Ansprüche 2 oder 3, dadurch gekennzeichnet, daß $R_5$ OH und $R_6$ H bedeutet.

5. Verwendung nach einem der Ansprüche 2 oder 3, dadurch gekennzeichnet, daß $R_5$ H und $R_6$ OH bedeutet.

6. Verwendung nach einem der Ansprüche 1 bis 5, dadurch gekennzeichnet, daß für die Verabreichung auf peroralem Weg die Pygnogenole der Formel (I) mit einem Anthrachinonderivat, insbesondere 1,8-Dihydroxyanthrachinon, kombiniert werden.

7. Verfahren zur Bestimmung der entzündungshemmenden Wirksamkeit in einem Pflanzenextrakt, das hier kennzeichnet, daß es darin besteht, die Pygnogenole der Formel (I) in diesem Extrakt quantitativ zu bestimmen.